# EUROPÄISCHE PATENTSCHRIFT

(11) **EP 1 974 047 B1**
(45) Veröffentlichungstag und Bekanntmachung des Hinweises auf die Patenterteilung: **01.09.2010**
(21) Anmeldenummer: 07700120.4
(22) Anmeldetag: 18.01.2007
(51) Int. Cl.: C12P 17/18, C12N 9/10

(54) **VERFAHREN ZUR REGIOSELEKTIVEN N(5)-FORMYLIERUNG VON (6S)-5,6,7,8-TETRAHYDROPTEROINSÄURE UND DERIVATEN DAVON**
PROCESS FOR REGIOSELECTIVE N(5)-FORMYLATION OF (6S)-5,6,7,8-TETRAHYDRO-PTEROIC ACID AND DERIVATIVES THEREOF
PROCEDE DE FORMYLATION N(5) REGIOSELECTIVE DE L'ACIDE (6S)-5,6,7,8-TETRAHYDROPTEROIQUE ET DE SES DERIVES

(30) Priorität: 19.01.2006 CH 83062006
(43) Veröffentlichungstag der Anmeldung: 01.10.2008
(73) Patentinhaber: Cerbios-Pharma S.A., CH-6917 Barbengo (CH)
(72) Erfinder: ARINI, Achille, CH-6922 Morcote (CH); COPPOLECCHIA, Raffaela, CH-6922 Morcote (CH); TOGNINI, Antonio, CH-6919 Carabietta (CH); MANZOTTI, Raffaela, 20047 Brugherio (MI) (IT); MARAZZA, Fabrizio, CH-6986 Novaggio (CH)
(74) Vertreter: Pozzi, Andrea
(86) Internationale Anmeldenummer: PCT/CH2007/000022
(87) Internationale Veröffentlichungsnummer: WO 2007/082402

(56) Entgegenhaltungen:
- EP-A1- 0 432 441
- WO-A-88/08844
- WO-A-92/02241
- GB-A- 721 742
- US-A- 2 824 045
- US-A- 5 198 547
- MACKENZIE R E ET AL: "Tetrahydropteroylpolyglutamate derivatives as substrates of two multifunctional proteins with folate-dependent enzyme activities." BIOCHIMICA ET BIOPHYSICA ACTA 11 JAN 1980, Bd. 611, Nr. 1, 11. Januar 1980 (1980-01-11), Seiten 187-195, XP002448347 ISSN: 0006-3002

## Beschreibung

Die vorliegende Erfindung betrifft ein Verfahren zur regioselektiven N(5)-Formylierung von (6S)-5,6,7,8-Tetrahydropteroinsäure und Derivaten davon, welche in Formel I definiert sind.

Die Formylierung von 5,6,7,8-Tetrahydrofolsäure, abgekürzt mit THF, ist ein wichtiges industrielles Verfahren und führt zu N(5)-Formyl-5,6,7,8-Tetrahydrofolsäure, auch Folinsäure oder Leucovorin genannt. Diese Verbindung ist ein pharmazeutisch aktiver Bestandteil, welcher im onkologischen und vitaminischen Bereich breit verwendet wird; siehe zum Beispiel "Folates and Pterins", R. L. Blakley, V. M. Whitehead Ed., Vol. 1-3, (1986), John Wiley & Sons.

Reagenzien, die für Ausführung dieser Transformation verwendet werden, umfassen Formamid und/oder Ameisensäure; siehe zum Beispiel das norwegische Patent Nr. NO 172 492 und das US Patent Nr. 5 198 547.

Gemäss der modernen Tendenz, umweltbelastende und toxische Reagenzien zu vermeiden, besteht der Bedarf, eine sicherere Alternative für die Formylierung von THF und von anderen Pteroinsäure-Derivaten zu finden.

Ein anderer Grund für das Auffinden einer Alternative zur Verwendung von Formamid und/oder Ameisensäure für die oben genannte Formylierung besteht darin, dass die Verwendung dieser "aggressiven" Reagenzien zur Bildung von verschiedenen unerwünschten Nebenprodukten führt: das isolierte Rohprodukt, zum Beispiel Folinsäure, muss daher mittels einer langen und komplexen Serie von Schritten gereinigt werden, um den hohen Reinheitskriterien zu entsprechen, die heutzutage für einen pharmazeutischen Aktivstoff verlangt werden; siehe wieder das oben genannte norwegische Patent Nr. NO 172 492.

Gemäss der allgemeinen Klassifizierung durch die Enzymkommission (EC), umfassend die EC Nummer und den Namen, gehört das Enzym, welches die Addition einer Formylgruppe an die N(5)-Stelle einer Verbindung der Formel I Katalysiert, zur Klasse der Transferasen und entspricht der Code-Nummer EC 2.1.2.5. Mehrere Definitionen, welche diesem Enzym zugeordnet sind, gehören immer noch der gegenwärtigen.Nomenklatur an und werden wie folgt zitiert:
Glutamat-formimidoyltransferase;
Glutamat-formyltransferase;
Formiminoglutaminsäure-transferase;
Formiminoglutaminsäure-formiminotransferase;
Glutamat-förmiminotransferase;
5-Formimidoyltetrahydrofolat: L-Glutamat-N-formimidoyltransferase.
Wichtig ist die Code-Nummer EC 2.1.2.5.
In der Natur ist Glutamat-formyltransferase, abgekürzt mit GFT, sowohl als Bestandteil des biochemischen Abbauweges von Histidin als auch im "C₁ Stoffwechsel durch Folat" wirksam.

In Säugetieren ist GFT als ein bifunktionelles Enzym vorhanden, da es neben der Formyltransferase Aktivität auch die Cyclodesaminierung von N(5)-Formimino-THF katalysiert, indem es diese Verbindung zu N(5),N'(10)-Methenyl-THF umwandelt.

Alle in der Literatur vorhandenen Studien über das EC 2.1.2.5 Enzym sind mit dem aus der Säugetierleber gewonnenen bifunktionellen Enzym durchgeführt worden. Diese Studien beziehen sich sowohl auf die Hauptaktivität als Formimino-transferase als auch auf die Minderaktivität als Formyl-transferase (Miller A. und Waelsch H., J. Biol. Chem. 1957, Vol. 228, Seiten 397-417; Tabor H. und Wyngarden, L. J. Biol. Chem. 1959, Vol. 234, Seiten 1830-1849; Silverman et al. J. Biol. Chem. 1957, Vol. 226, Seiten 83-84; Bortoluzzi und McKenzie J. Biol. Chem. 1983, Vol. 61, Seiten 248-253).

Nach der Klonierung und Sequenzierung des aus der Schweineleber gewonnenen GFT Enzyms (Murley L.L. et. al, Journal of Biol. Chem. 1993. Vol 268, Seiten 22820-22824) sind andere DNA Sequenzen gefunden worden, die für mutmassliche Formimino-transferasen kodieren könnten. Diese DNA Sequenzen sind auch in anderen Organismen als in Säugetieren gefunden worden. Mit den neuesten Fortschritten in der automatischen Sequenzierung hat man tatsächlich eine grosse Anzahl an genomischen Daten von relativ zahlreichen Prokaryoten und Eukaryoten gewinnen können.

Es sind auch Berichte über Pflanzenenzyme mit Glutaminsäure-formimino-transferase Aktivität veröffentlicht worden, die sich auf Arbeiten über *Oryza sativa, Triticum aestivum und Glycine max* (siehe die US Patent Anmeldung Nr. 2002/0102689) beziehen.

Aufgrund der Literaturangaben, die die Funktionalität des aus Schweineleber gewonnenen GFT Enzyms nachweisen, wäre es sicher naheliegender gewesen, die entsprechende GFT-kodierende DNA Sequenz zu klonieren und somit das mit hoher Wahrscheinlichkeit funktionsfähige Enzym zu erhalten. Da das Leberenzym jedoch bifunktionell ist, wäre es nicht geeignet für die industrielle Produktion der (6S)-N(5)-formylierten Verbindung der Formel I, insbesondere weil die enzymatische Reaktion als Endprodukt die N(5),N(10)-Methenyl- anstatt der N(5)-Formylformergeben hätte.

Es ist ein Ziel der vorliegenden Erfindung, ein "milderes" und umweltfreundliches Formyierungsverfahren zu Verfügung zu stellen, welches die Bildung der meisten beobachteten Nebenprodukte vermeidet.

Dieses Verfahren soll ein Rohprodukt, insbesondere Folinsäure, ergeben, welches ein viel kürzeres Reinigungsverfahren benötigt, und eine höhere Gesamtausbeute an reinem Produkt, insbesondere (6S)-N(5)-Formyl-THF, ergibt.

Dieses Verfahren soll eine enzymatische Alternative zum oben genannten rein chemischen Verfahren beinhalten.

Dieses Verfahren soll für die industrielle Herstellung von (6S)-N(5)-formylierten Verbindungen der Formel II geeignet sein.

US 5 198 547 offenbart ein Verfahren zur Herstellung von N5-Formyl-(6S)-Tetrahydrofolsäure durch Formilierung von (6S)-Tetrahydrofolsäure: wie oben erwähnt, werden für die Formilierung aggressive chemische Reagenzien verwendet.

WO 92/02241 A offenbart ein nicht-enzymatisches Verfahren zur Herstellung von reinem N5-Formyl-(6S)-Tetrahydrofolsäure-Polyglutamat.

EP 0 432 441 A1 offenbart ein Verfahren zur Herstellung von reinem N5-Formyl-(6S)-Tetrahydrofolsäure durch Verwendung eines Tetrahydrofolatformylase umfassenden Enzyms.

WO 88/08844 A offenbart ein Verfahren zur Trennung von (6R)- und (6S)-Folinsäure durch Umkristallisation von Erdalkalisalzen der (6R,S)-Folinsäure.

In der vorliegenden Erfindung soll ein rekombinantes Enzym prokaryotischer bakterieller Herkunft, das ausschliesslich die Formimino (formyl) transferase Funktion aufweist und das somit für die industrielle Produktion der N(5)-formylierten Verbindung der Formel I geeignet ist, verwendet werden.

Mit der vorliegenden Erfindung werden die obigen ziele erreicht.

Das erfindungsgemässe Verfahren zur regioselektiven N(5)-Formyl-ierung von (6S)-5,6,7,8-Tetrahydropteroinsäure und Derivaten davon, welche in Formel I definiert sind, ist dadurch gekennzeichnet, dass man
- zu einem Gemisch aus einer Verbindung der Formel I, einem Antioxidationsmittel und einem Formyldonator in einem Reaktionsgefäss eine wässrige Pufferlösung mit einem pH Wert von 6,5 bis 7,5 hinzugibt,
- durch die erhaltene Suspension ein Inertgas durchbläst,
- dann tropfenweise oder portionenweise unter ständigem Rühren eine wässrige Lösung einer Base hinzugibt, bis eine klare Lösung entsteht, welche einen pH Wert zwischen 6,5 bis 8,3 aufweist,
- dann eine rekombinante monofunktionelle Glutamat-Formyltransferase, abgekürzt mit rGET, welche nur die Funktion der Forminino-(Formyl-)transferase zeigt, d.h. das Enzym EC 2.1.2.5, in einer wässrigen, weiter oben definierten Pufferlösung hinzugibt,
- dann die Temperatur auf einen Bereich von 30°C bis 45°C erhöht und das Reaktionsgemisch reagieren lässt, bis wenigstens 95% des Formyltransfers vom Formyl-donator auf die Verbindung der Formel I erfolgt ist,
- dann das erhaltene Reaktionsgemisch auf eine Temperatur von 0°C bis 5°C abkühlt,
- dann eine wässrige Lösung einer Mineralsäure tropfenweise hinzugibt, bis ein pH Wert von 2,7 bis 3,1 erreicht ist, wobei die rohe Verbindung der Formel II ausfällt,
- das Rühren während wenigstens 1 Stunde fortsetzt, um die oben genannte Fällung zu vervollständigen, und
- die rohe Verbindung der Formel II gewinnt.

Bevorzugte Ausführungsformen dieser Erfindung sind in den abhängigen Ansprüchen definiert.

Im folgenden Teil werden mögliche Ausführungsformen der vorliegenden Erfindung beschrieben.

Dabei wird Bezug auf die Figuren genommen.

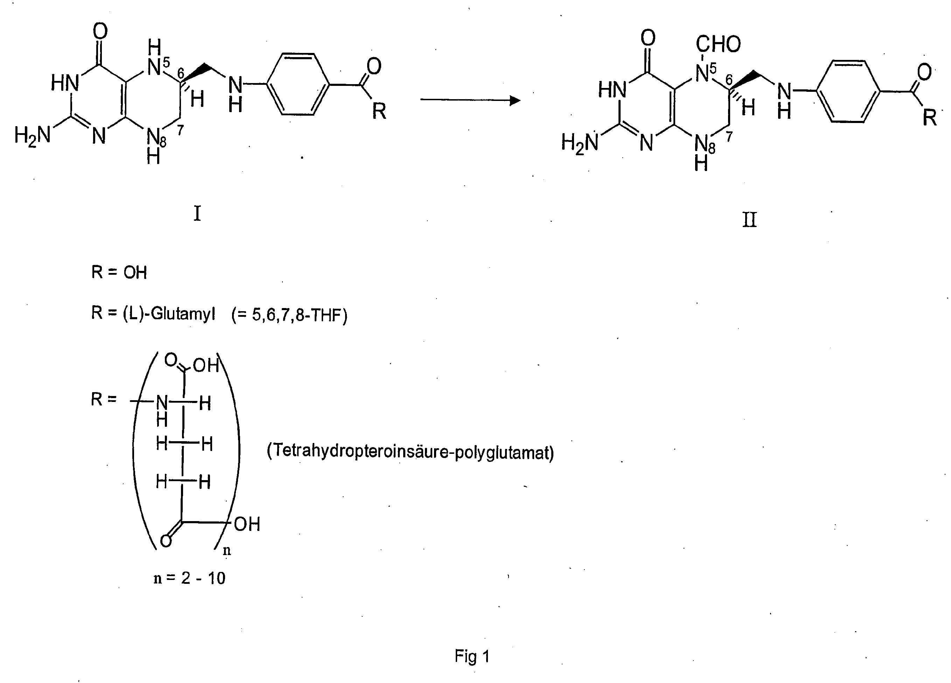

Figur 1 zeigt das Reaktionsschema, worin auch der Rest R definiert ist.

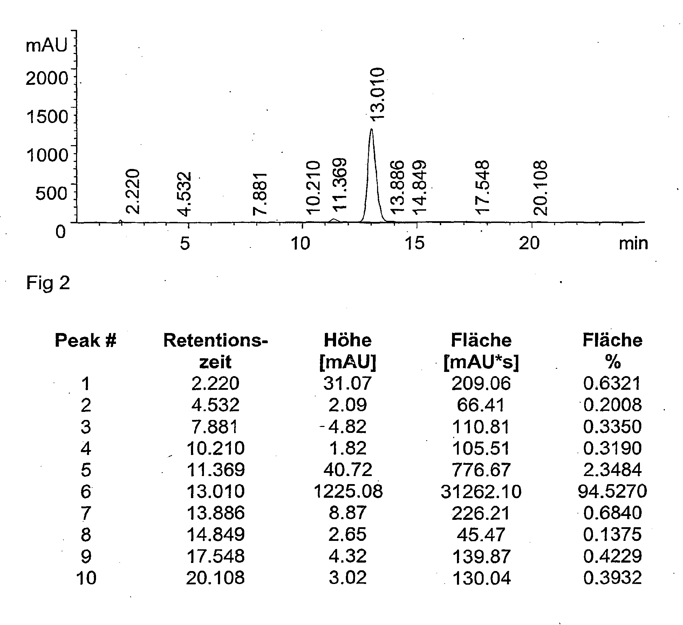

Figur 2 zeigt ein HPLC Chromatogramm an einer "reverse phase" Säule von roher (6S)-N(5)-Formyl-THF, hergestellt gemäss dem norwegischen Patent Nr. NO 172 492.
Figur 3A zeigt ein HPLC Chromatogramm an einer "reverse phase" Säule von roher (6S)-N(5)-Formyl-THF, hergestellt gemäss Beispiel 2 der vorliegenden Erfindung. Es ist festgehalten, dass der grosse Peak bei der Retensionszeit 1. min Ascorbinsäure entspricht.
Figur 3B zeigt ein HPLC Chromatogramm an einer "reverse phase" Säule von gereinigter (6S)-N(5)-Formyl-THF, hergestellt gemäss Beispiel 2 der vorliegenden Erfindung, nach einer Resuspension in kaltem Wasser und einer Filtration.

In der vorliegenden Erfindung wird ein rekombinantes monofunktionelles Enzym prokaryotischen, bakteriellen Ursprungs verwendet, welches nur die Funktion der Formimino (Formyl) Transferase zeigt.

Dieses Enzym ist nicht das gleiche wie das oben erwähnte bifunktionelle Leberenzym, aber es ist in Teilen ähnlich, insbesondere im Teil, welcher der Formimino Transferase Domäne entspricht.

Der Vorteil der Verwendung eines aus einem Mikroorganismus gewonnenen prokaryotischen, bakteriellen Enzyms, und insbesondere des von *Streptococcus pyogenes* kodierten Enzyms, besteht in seiner monofunktionellen Glutamat Formimino (Formyl) Transferase Aktivität.

Die monofunktionelle Eigenschaft dieses Enzyms liess sich durch die vergleichende Analyse seiner Aminosäuresequenz in der genomischen Datenbank herleiten, obwohl keine Voraussage über seine tatsächliche Aktivität getroffen werden konnte, bevor die biochemische Reaktion durchgeführt wurde.

Die in der vorliegenden Erfindung verwendete DNA-Sequenz und die Aminosäuresequenz von GFT sind verschieden von den pflanzlichen Sequenzen, die in der oben genannten US Patentanmeldung Nr. 2002/0102689 beschrieben wurden.

Bedingt durch die inhärente Stereoselektivität des Enzyms resultiert die Verwendung von (6RS)-5,6,7,8-Tetrahydrofolsäure als Substrat in der ausschliesslichen Formylierung des (6S)-Isomers.

Die folgenden Beispiele illustrieren die vorliegende Erfindung.

### Beispiel 1

### Herkunft des S. pyogenes Glutamat-formyl-transferase (GFT) Gens

Das Gen, das für GFT von *S. pyogenes* (ATCC 19615) kodiert, wurde in *Escherichia coli* (*E. coli*) Stamm BL21 (DE) kloniert und überexprimiert.

Auf diese Weise wurde genügend rekombinantes monofunktionelles Enzym hergestellt.

### Extraktion der genomischen DNA

25 ml einer Kultur von *S. pyogenes* ATCC 19615 wurden über Nacht in MRS Brühe (Oxoid) bei einer Temperatur von 37°C gezüchtet.

Das nach der Zentrifugation erhaltene Pellet wurde anhand einer Verfahrensanleitung weiter verarbeitet, die im Vergleich zu derjenigen im Kit für die Extraktion genomischer DNA (Qiagen) modifiziert wurde.

Das Pellet wurde in 500 µl STET Puffer (8% Saccharose, 50 mM Tris-Cl pH 8,0, 50 mM EDTA, 0,1% Triton X-100) in Lösung gebracht und 400 µl einer Lysozymlösung (50 mg/ml) wurden hinzugefügt. Das Gemisch wurde bei einer Temperatur von 37°C während 150 Minuten bis zur vollständigen Lyse der Zellen inkubiert.

Das lysierte Gemisch wurde gemäss der Anleitung des Kit-Herstellers (Qiagen) weiter verarbeitet, mit der Ausnahme, dass nur die Hälfte der angegebenen Volumina verwendet wurde.

Nach der Isopropanol-Präzipitierung wurde die erhaltene DNA ein zweites Mal gereinigt, indem zuerst eine Behandlung mit Phenol/Chloroform (1:1), dann mit Chloroform und schliesslich eine Ethanol-Präzipitierung eingesetzt wurden.

### GFT DNA Isolierung und Amplifizierung

Genomische DNA aus *S. pyogenes* ATCC 19615 wurde für die Polymerase-Ketten-Reaktion (PCR) verwendet, um die GFT DNA Sequenz zu amplifizieren, wobei das folgende Oligonukleotidpaar bei einer Hybridisierungstemperatur von 45°C eingesetzt wurde:
Oligo 1:
   5' AGGAGGAAATAAAAATGGCGAAAATTGTTGAATGTATTCCC 3'
Oligo 2:
   5' TAAGAATTCCTACTAACCTAGCAAATGGTTTTCAAGAATC 3'

Die Klonierung und die anschliessende Sequenzierung der kodierenden Region ergaben die folgende Sequenz:

ATGGCGAAAATTGTTGAATGTATTCCCAACTTCTCAGAAGGCCAAAATCAGGCTGTTATC
GATGGTTTGGTAGCGACGGCCAAAAGTATTCCTGGAGTGACCCTTCTCGACTACTCTTCT
GATGCGAGCCACAACCGGAGCGTGTTTACCTTGGTTGGAGATGACCAGTCTATTCAAGAA
GCAGCTTTCCAACTGGTGAAATACGCTTCTGAAAACATTGATATGACCAAACATCATGGC
GAACACCCTCGTATGGGAGCAACTGATGTCTGCCCATTCGTTCCGATCAAAGACATCACT
ACACAAGAATGTGTTGAGATTTCCAAACAAGTCGCAGAACGGATTAACCGTGAACTTGAC
ATTCCAATCTTCCTTTATGAAGATTCTGCCACACGTCCAGAACGTCAAAACCTTGCTAAA
ATCCGTAAAGGTCAGTTTGAAGGCATGCCAGAAAAACTGTTGGAAGAGGACTGGGCACCT
GACTACGGAGATCGTAAGATTCACCCAACTGCTGGGGTAACTGCCGTTGGTGCTCGCATG
CCATTGGTTGCCTTCAATGTTAATTTGGATACTGACAATATCGACATTGCTCATAAAATT
GCTAAAATTATCCGCGGTTCAGGTGGTGGCTACAAATATTGTAAGGCTATCGGTGTCATG
TTAGAAGACCGTCATATTGCCCAAGTCTCCATGAACATGGTTAACTTTGAAAAATGTTCT
CTTTACCGTACTTTTGAAACCATCAAATTTGAAGCGCGTCGCTACGGTGTGAATGTTATT
GGTTCTGAAGTCATCGGCTTAGCGCCAGCCAAGGCCTTAATCGATGTGGCTGAATACTAC
TTACAAGTTGAGGACTTTGACTACCCTAAACAGGTTCTTGAAAACCATTTGCTAGGTTAG

### Klonierung in den Expressionsvektor pET22b

Um eine hohe Ausbeute hinsichtlich Expression und Reinigung des rekombinanten monofunktionellen rGFT zu erhalten, wurde eine Sequenz, die für 6 Histidine (His-tag) kodiert, an das carboxylterminale Ende des rGFT angefügt.

Dies wurde erhalten, indem das rGFT DNA Fragment in die XbaI/BamHI Restriktionsstelle des kommerziellen Expressionsvektors pET22b (Novagen, EMD Biosciences) hinein kloniert wurde.

Zuerst wurden die XbaI/BamHI Restriktionsstellen ins rGFT DNA Fragment hineingeführt, um dieselbe Sequenz mit dem "His-Tag" im Expressionsvektor fusionieren zu können. Dies wurde erhalten, indem rGFT DNA mit dem folgenden Oligonukleotidpaar bei einer Hybridisierungstemperatur von 50°C amplifiziert wurde:
Oligo 3:
   5' CCCTCTAGAAGGAGGAAATAAAAATGGCG 3'
Oligo 4:
   5'GTCCCAGGATCCAAACCTAGCAAATGGTTTTCAAGAATCTG 3'

Dann wurde das neu amplifizierte rGFT DNA Fragment in die XbaI/BamHI Restriktionsstellen von pET22b kloniert, und das klonierte Konstrukt wurde in *E. coli* BL21 (DE) transformiert.

Das nach der Klonierung in *E. coli* BL21 (DE) erhaltene Plasmid erlaubt die Expression des rGFT Gens (mit dem C-terminalen His-tag), das unter der Kontrolle des T7 Promoters steht.

Die Expression des so modifizierten Enzyms wurde durch Western-Blotting unter Verwendung von anti-His Antikörpern (Novagen) überprüft.

### Züchtung von rGFT-Enzym exprimierendem E. coli BL21 (DE)

Eine Kultur von rekombinantem *E. coli* BL21 (DE), welches das rGFT Enzym exprimiert, wurde über Nacht gezüchtet, dann 1:50 in 300 ml frischer Luria Brühe (LB) verdünnt und in Gegenwart von Ampicillin (100 µg/ml) bei einer Temperatur von 37°C in einem Schüttler (180 rpm) bis zum Erreichen einer OD₆₀₀ von 0,7 gezüchtet.

Nach Induzierung mit 1,4 ml von 0,1 M IPTG (Isopropyl-thio-galaktosid) wurde die Inkubation für etwa 3 Stunden fortgesetzt.

### Reinigung von rGFT

Die erhaltene Zellkultur wurde abzentrifugiert, und der Überstand wurde verworfen.

Das Zellpellet wurde bei einer Temperatur von -70°C aufbewahrt.

Das Zellpellet wurde dann in 30 ml Puffer A (20 mM Natriumphosphat, 150 mM NaCl, 10 mM MgCl₂, 1 mM Phenylmethylsulphonylfluörid; pH 7,4) aufgenommen.

Das resuspendierte Zellpellet wurde dann für 30 Minuten bei einer Temperatur von 4°C in Gegenwart von 0,5 g/l Lysozym inkubiert.

Die erhaltene Suspension wurde in einem Mikrofluidizer bei einem Druck von 10'000 psi behandelt.

Dann wurde die Suspension während 45 Minuten bei einer Temperatur von 4°C mit DNase/RNase (jeweils 10 µg/ml) inkubiert.

Die Suspension wurde dann während 10 Minuten bei 10'000 rpm zentrifugiert, und das Pellet wurde verworfen.

Der Überstand wurde auf eine 5 ml Ni²⁺-Sepharose 6 FF Säule (Amersham Biosciences) aufgetragen.

Die Bindung und das Waschen des ungebundenen Probenmaterials wurden mit Puffer B (20 mM Natriumphosphat, 500 mM NaCl; pH 7,5) durchgeführt.

Die Eluierung des gebundenen rGFT erfolgte durch Puffer C (20 mM Natriumphosphat, 500 mM NaCl, 500 mM Imidazol; pH 7,5).

Für die endgültige Formulierung und Aufbewahrung wurde ein Pufferaustausch (Puffer D: 100 mM Kaliumphosphat; pH 7,0) durchgeführt, wobei eine 5 ml Sephadex G25-F Säule verwendet wurde.

Die Lösung mit dem rekombinanten monofunktionellen rGFT Enzym wurde bei einer Temperatur von -70°C gelagert.

Die Aktivität (Units/ml und Units/mg) einer Kontrollprobe wurde entsprechend dem Sigma-Test für das EC 2.1.2.5 Enzym gemessen, wobei Formyl-(L)-glutaminsäure anstelle von Formimino-(L)-glutaminsäure und eine Inkubation bei einer Temperatur von 37°C statt 25°C benutzt wurden.

Der Sigma-Test ist eine Modifizierung des in Tabor H. und Wyngarden L., Journal of Clinical Investigation, 1958, Vol. 37, Seiten 824-828 beschriebenen Tests.

### Beispiel 2

445 mg (1 mMol) (6S)-THF, hergestellt gemäss EP 0 600 460 B1, 880 mg (5 mMol) Ascorbinsäure und 875 mg (5 mMol) N-Formyl-(L)-glutaminsäure wurden in einen 25 ml Dreihalskolben gegeben, welcher mit einem Magnetrührer, einem Stickstoffeinleitungsrohr und einem Thermometer ausgerüstet war.

N-Formyl-(L)-glutaminsäure wurde nach der Vorschrift von H. Tabor, A. H. Mehler, J. Biol. Chem., 210, 559 (1954) hergestellt.

Zum oben beschriebenen Gemisch wurden unter Rühren und einer Stickstoffatmosphäre 5 ml eines 100 mM Kaliumphosphat Puffers zugegeben, welcher einen pH Wert von 6,7 hatte.

Zur erhaltenen Suspension wurde tropfenweise 20%-ige NaOH solange zugegeben, bis eine homogene Lösung erhalten wurde, welche einen pH Wert von 6,7 hatte.

Um Spuren von Sauerstoff zu entfernen, wurde dann das Reaktionsgefäss evakuiert und mit Stickstoff gespült; diese Prozedur wurde noch zweimal wiederholt.

Dann wurden 0,35 U der rekombinanten monofunktionellen Glutamat-formyltransferase, gelöst in 0,32 ml eines 100 mM Kaliumphosphat Puffers bei pH 6,7, zugegeben. Dieses Enzym wurde gemäss dem oben beschriebenen Beispiel 1 hergestellt.

Dann wurden erneut 3 Zyklen des Evakuieren und der Stickstoffspülung durchgeführt.

Die Temperatur dieses Reaktionsgemisches wurde auf 40°C erhöht und während insgesamt 72 Stunden gerührt.

Der Fortschritt der Formylierungsreaktion wurde mittels HPLC unter Verwendung einer "reverse phase" Säule verfolgt.

Während der oben erwähnten 72-stündigen Reaktionszeit wurden noch zusätzliche 100 mg von N-Formyl-(L)-glutaminsäure (0,57 mMol) in 4 Portionen zugegeben.

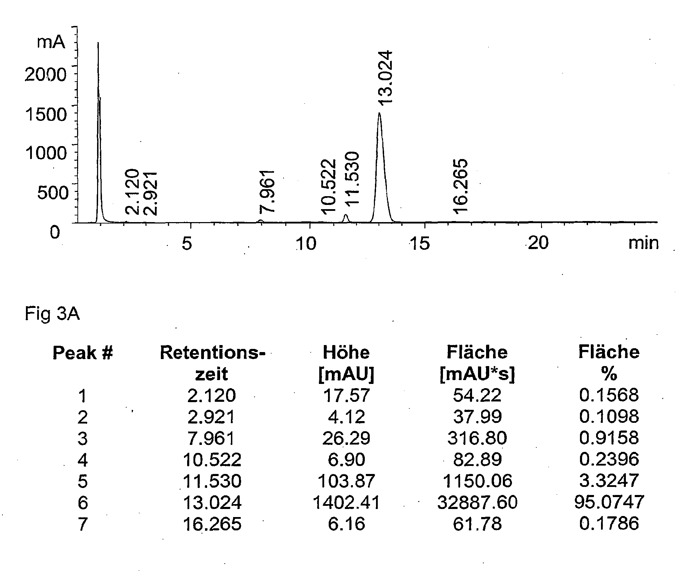

Das am Schluss der Reaktion durchgeführte HPLC Chromatogramm zeigte, dass die erhaltene (6S)-N(5)-Formyl-THF eine Reinheit von 95,07% hatte. Siehe die Figur 3A.

Dann wurde das Reaktionsgemisch auf eine Temperatur von 5°C abgekühlt.

Unter Rühren wurde 18%-ige HCl zugegeben, bis ein pH Wert von 3,0 erreicht wurde.

Während der Säurezugabe entstand ein weisser Festkörper.

Um die Fällung zu vervollständigen wurde die erhaltene Suspension während einer weiteren Stunde bei einer Temperatur von 5°C gerührt.

Der erhaltene Festkörper wurde mittels Filtration isoliert, dann in 2 ml kaltem Wasser resuspendiert und erneut filtriert.

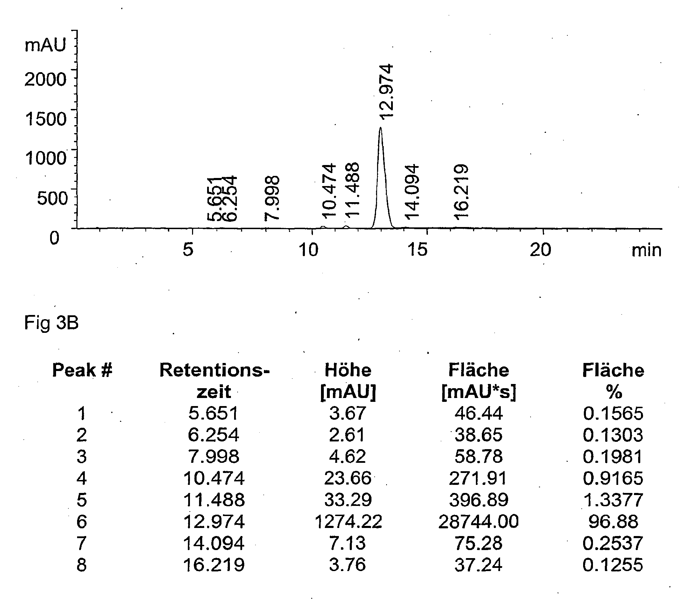

Die so erhaltene feste (6S)-N(5)-Formyl-THF zeigte im HPLC Chromatogramm eine Reinheit von 96,88%. Siehe die Figur 3B.

Das Produkt kann zusätzlich gereinigt werden, indem man zum Beispiel das entsprechende Ca²⁺-Salz herstellt, wie das im norwegischen Patent Nr. NO 172 492 beschrieben ist.

## Patentansprüche

1. Verfahren zur regioselektiven N(5)-Formylierung von (6S)-5,6,7,8-Tetrahydropteroinsäure und Derivaten davon, welche in Formel I 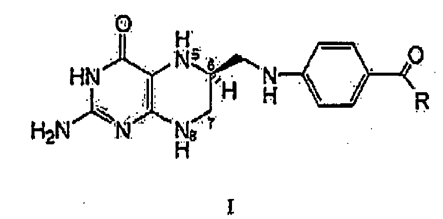 R = OH
R = (L)-glutamyl (= 5,6,7,8-THF) 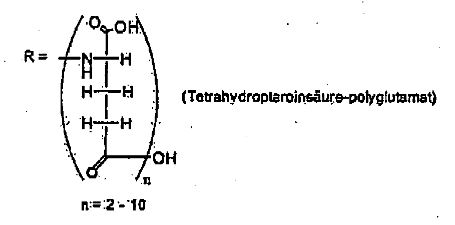 definiert sind, **dadurch gekennzeichnet, dass** man
- zu einem Gemisch aus einer Verbindung der Formel I, einem Antioxidationsmittel und einem Formyl-donator in einem Reaktionsgefäss eine wässrige Pufferlösung mit einem pH Wert von 6,5 bis 7,5 hinzugibt,
- durch die erhaltene Suspension ein Inertgas durchbläst,
- dann tropfenweise oder portionenweise unter ständigem Rühren eine wässrige Lösung einer Base hinzugibt, bis eine klare Lösung entsteht, welche einen pH Wert zwischen 6,5 bis 8,3 aufweist,
- dann eine rekombinante monofunktionelle Glutamat-Formyltransferase, abgekürzt mit rGFT, welche nur die Funktion der Formimino-(Formyl-) transferase zeigt, d.h. das Enzym EC 2.1.2.5, in einer wässrigen, weiter oben definierten Pufferlösung hinzugibt,
- dann die Temperatur auf einen Bereich von 30°C bis 45°C erhöht und das Reaktionsgemisch reagieren lässt, bis wenigstens 95% des Formyltransfers vom Formyl-donator auf die Verbindung der Formel I erfolgt ist,
- dann das erhaltene Reaktionsgemisch auf eine Temperatur von 0°C bis 5°C abkühlt,
- dann eine wässrige Lösung einer Mineralsäure tropfenweise hinzugibt, bis ein pH Wert von 2,7 bis 3,1 erreicht ist, wobei die rohe Verbindung der Formel II 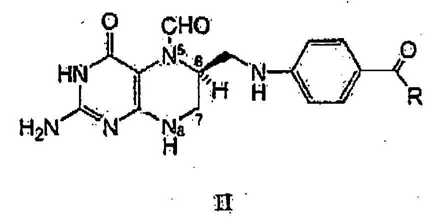 wo R das gleiche Radikal wie in Formel I ist, ausfällt,
- das Rühren während wenigstens 1 Stunde fortsetzt, um die oben genannte Fällung zu vervollständigen, und
- die rohe Verbindung der Formel II gewinnt.

2. Verfahren nach Anspruch 1, **dadurch gekennzeichnet, dass** die Verbindung der Formel I (65)-5,6,7,8-Tetrahydrofolsäure ist, abgekürzt mit (6S)-THF.

3. Verfahren nach einem der Ansprüche 1 bis 2, **dadurch gekennzeichnet, dass** man in das genannte Reaktionsgefäss zuerst die Verbindung der Formel I in fester Form hinzugibt,
dann das Antioxidationsmittel hinzugibt, vorzugsweise ausgewählt aus der Gruppe, bestehend aus Ascorbinsäure, Mercaptoethanol und Dithiotreithol, und
dann den Formyl-donator hinzugibt, vorzugsweise ausgewählt aus der Gruppe, bestehend aus N-Formyl-(L)-glutaminsäure, N-Formyl-(L)-glutamin, N-Formyl-(L)-isoglutamin und N-Formyl-(L)-asparaginsäure, oder deren wasserlösliche Salze, vorzugsweise die Natriumsalze oder die Kaliumsalze, und
dass man das Gemisch bei Raumtemperatur und unter einem Inertgas herstellt.

4. Verfahren nach einem der Ansprüche 1 bis 3, **dadurch gekennzeichnet, dass** die wässrige Pufferlösung mit einem pH Wert von 6,5 bis 7,5 ein Phosphatpuffer ist, vorzugsweise ein 10 mM bis 1 M, insbesondere 100 mM, Natrium-oder Kaliumphosphatpuffer.

5. Verfahren nach einem der Ansprüche 1 bis 4, **dadurch gekennzeichnet, dass** die wässrige Lösung einer Base eine 20% NaOH Lösung oder eine 20% KOH Lösung ist.

6. Verfahren nach einem der Ansprüche 1 bis 5, **dadurch gekennzeichnet, dass** die klare Lösung, welche nach der Hinzugabe der Base erhalten wird, einen pH Wert von 6,5 bis 6,8 aufweist.

7. Verfahren nach einem der Ansprüche 1 bis 6, **dadurch gekennzeichnet, dass** nach der Hinzugabe des Enzyms die Temperatur auf einen Bereich von 37°C bis 42°C erhöht wird.

8. Verfahren nach einem der Ansprüche 1 bis 7, **dadurch gekennzeichnet, dass** man nach der Hinzugabe des Enzyms die Komponenten während wenigstens 8 Stunden, vorzugsweise während 1 bis 5 Tag(en) reagieren lässt.

9. Verfahren nach einem der Ansprüche 1 bis 8, **dadurch gekennzeichnet, dass** die Mineralsäure HCl, vorzugsweise 18% HCl, oder H₂SO₄ ist.

10. Verfahren nach einem der Ansprüche 1 bis 9, **dadurch gekennzeichnet, dass** das Rühren für die Vervollständigung der Fällung der rohen Verbindung der Formel II während wenigstens 2 Stunden erfolgt.

11. Verfahren nach einem der Ansprüche 1 bis 10, **dadurch gekennzeichnet, dass** die rohe Verbindung der Formel II mittels Filtration oder mittels Zentrifugation gewonnen wird.

12. Verfahren nach einem der Ansprüche 1 bis 11, **dadurch gekennzeichnet, dass** das molare Verhältnis zwischen dem Formyl-donator und der Verbindung der Formel I während der Formylierungsreaktion im Bereich von 2:1 bis 10:1, insbesondere von 5:1 bis 3:1, gehalten wird.

13. Verfahren nach einem der Ansprüche 1 bis 12, **dadurch gekennzeichnet, dass** das molare Verhältnis zwischen dem Antioxidationsmittel und der Verbindung der Formel I im Bereich von 1:1 bis 5:1, insbesondere von 2:1 bis 3:1, ist.

14. Verfahren nach einem der Ansprüche 1 bis 13, **dadurch gekennzeichnet, dass** eine Menge von 0,01 bis 0,1 U/ml, insbesondere von 0,03 bis 0,04 U/ml, an Enzym von 1 mM bis 200 mM an (65)-THF in einer Zeit von 24 Stunden bis 48 Stunden bei einer Temperatur von 37°C bis 42°C und einem pH Wert von 6,5 bis 6,8 formyliert.

15. Verfahren nach einem der Ansprüche 1 bis 14, **dadurch gekennzeichnet, dass** die weitere Reinigung der rohen Verbindung der Formel II eine Resuspension des rohen Gemisches in kaltem Wasser und eine Filtration beinhaltet.

16. Verfahren nach einem der Ansprüche 1 bis 15, **dadurch gekennzeichnet, dass** die genomische DNA, welche für das genannte rekombinante monofunktionelle Enzym ködiert, entweder aus einem Mikroorganismus, insbesondere aus der Familie der Streptococcaceae, vorzugsweise aus der S.pyogenes Art, zum Beispiel aus dem Stamm ATCC 19615, oder aus einem mutanten Stamm mit der gleichen Funktion, isoliert wird, oder chemisch hergestellt wird.

17. Verfahren nach einem der Ansprüche 1 bis 16, **dadurch gekennzeichnet, dass** das genannte rekombinante monofunktionelle Enzym in einer immobilisierten Form auf einem. Träger vorliegt, zum Beispiel auf einem Epoxy aktivierten Harz.

18. Verwendung einer rekombinanten monofunctionellen Glutamat-Formyltransferase welche nur die Funktion der Forminino-(Formyl-) transferase zeight, d.h. das Enzym EC 2.1.2.5, für die regioselektive N(5)-Fomlylierung von (6S)-5,6,7,8-Tettahydropteroinsäure und Derivaten davon, welche in Formel I 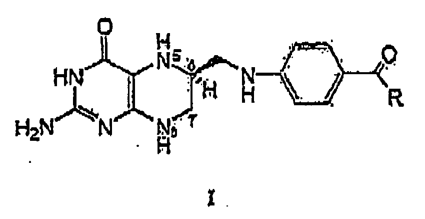 R = OH
R = (L)-glutamyl (= 5,6,7,8-THF) 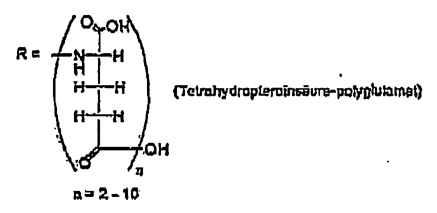 definiert sind.

## Claims

1. Method for regioselective N(5)-formylation of (6S)-5,6,7,8-tetrahydropteroic acid and derivatives thereof, which are defined in Formula I 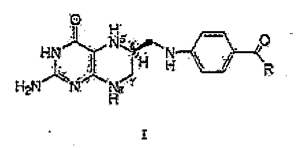 R = OH
R + (L)-glutamyl (= 5,6,7,8-THF) 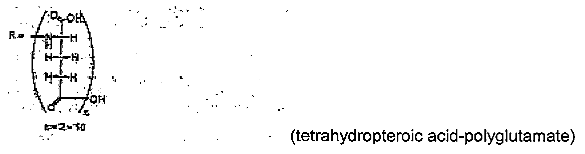 **characterised in that**
- an aqueous buffer solution with a pH value of 6.5 to 7.5 is added to a mixture containing a compound of Formula I, an antioxidant, and a formyl donor in a reaction vessel,
- an inert gas is blown through the suspension obtained,
- then an aqueous solution of a base is added dropwise or in portions while continuously stirring until a clear solution is formed which has a pH value between 6.5 and 8.3,
- then a recombinant monofunctional glutamate-formyl transferase, abbreviated to rGFT, which only shows the formimino-(formyl-) transferase function, i.e. the enzyme EC 2.1.2.5, is added to an aqueous buffer solution, defined in more detail above,
- then the temperature is increased to a range of 30°C to 45 °C and the reaction mixture is left to react until there is at least 95% formyl transfer of formyl donor to the compound of Formula I,
- then the reaction mixture obtained is cooled down to a temperature of 0 °C to 5°C,
- then an aqueous solution of a mineral acid is added dropwise until a pH value of 2.7 to 3.1 is attained, whereby the raw compound of Formula II, 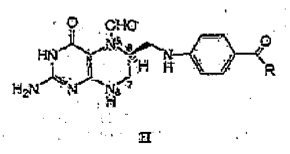 where R is the same radical as in Formula I, precipitates,
- stirring is continued for at least 1 hour in order to complete the aforementioned precipitation, and
- the raw compound of Formula II is obtained.

2. Method according to claim 1, **characterised in that** the compound of Formula I is (6S)-5,6,7,8-tetrahydrofolic acid, abbreviated as (6S)-THF.

3. Method according to any of claims 1 to 2, **characterised in that** the compound of Formula I in solid form is added first to the reaction vessel mentioned,
then the antioxidant is added, preferably selected from the group consisting of ascorbic acid, mercaptoethanol and dithiothreitol, and
then the formyl donor is added, preferably selected from the group consisting of N-formyl-(L)-glutamic acid, N-formyl-(L)-glutamine, N-formyl-(L)-isoglutamine, and N-formyl-(L)-asparagine acid, or their water-soluble salts, preferably the sodium salts or the potassium salts, and
the mixture is prepared at room temperature and under inert gas.

4. Method according to any of claims 1 to 3, **characterised in that** the aqueous buffer solution with a pH value of 6.5 to 7.5 is a phosphate buffer, preferably a 10 mM to 1 M, particularly 100 mM, sodium or potassium phosphate buffer.

5. Method according to any of claims 1 to 4, **characterised in that** the aqueous solution of a base is a 20% NaOH solution or a 20% KOH solution.

6. Method according to any of claims 1 to 5, **characterised in that** the clear solution, which is obtained after adding the base, has a pH value of 6.5 to 6.8.

7. Method according to any of claims 1 to 6, **characterised in that** after adding the enzyme the temperature is raised to a range of 37 °C to 42 °C.

8. Method according to any of claims 1 to 7, **characterised in that** after adding the enzyme the components are left to react for at least 8 hours, preferably for 1 to 5 day(s).

9. Method according to any of claims 1 to 8, **characterised in that** the mineral acid is HCl, preferably 18% HCl, or H₂SO₄.

10. Method according to any of claims 1 to 9, **characterised in that** stirring is carried out for at least 2 hours to complete the precipitation of the raw compound of Formula II.

11. Method according to any of claims 1 to 10, **characterised in that** the raw compound of Formula II is obtained by means of filtration or by means of centrifugation.

12. Method according to any of claims 1 to 11, **characterised in that** the molar ratio between the formyl donor and the compound of Formula I is maintained within the range of 2:1 to 10:1, particularly of 5:1 to 3:1, during the formylation reaction.

13. Method according to any of claims 1 to 12, **characterised in that** the molar ratio between the antioxidant and the compound of Formula I is within the range of 1:1 to 5:1, particularly of 2:1 to 3:1.

14. Method according to any of claims 1 to 13, **characterised in that** a quantity of 0.01 to 0.1 U/ml, particularly of 0.03 to 0.04 U/ml, of enzyme formylates 1 mM to 200 mM of (6S)-THF within a period of 24 hours to 48 hours at a temperature of 37°C to 42 °C and at a pH value of 6.5 to 6.8.

15. Method according to any of claims 1 to 14, **characterised in that** the further purification of the raw compound of Formula II comprises resuspending the raw mixture in cold water and filtering.

16. Method according to any of claims 1 to 15, **characterised in that** the genomic DNA, which codes for the recombinant monofunctional enzyme mentioned, is isolated either from a micro-organism, particularly from the Streptococcaceae family, preferably from the species S. pyogenes, for example from the stem ATCC 19615, or from a mutant stem with the same function, or is chemically manufactured.

17. Method according to any of claims 1 to 16, **characterised in that** the recombinant monofunctional enzyme mentioned is present in an immobilised form on a substrate, for example on an epoxy-activated resin.

18. Use of a recombinant monofunctional glutamate formyl transferase, which only shows the forminino-(formyl-)transferase function, i.e. the enzyme EC 2.1.2.5, for the regioselective N(5)-formylation of (6S)-5,6,7,8-tetrahydropteroic acid and derivatives thereof, which are defined in Formula I. 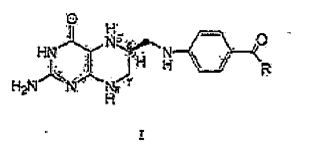 R = OH
R + (L)-glutamyl (= 5,6,7,8-THF)

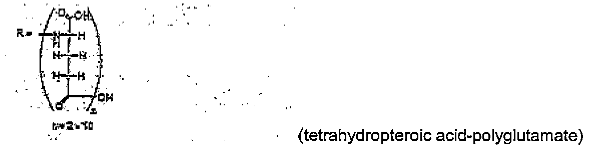

## Revendications

1. Procédé de N(5)-formylation régiosélective de l'acide (6S)-5,6,7,8-tétrahydroptéroïque et de ses dérivés, qui sont définis par la formule (I) : 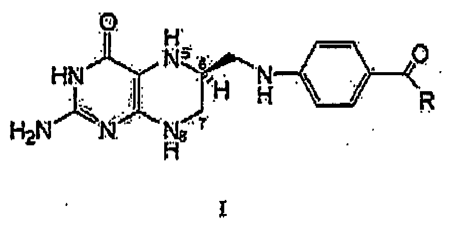 R = OH
R = (L)-glutamyle (= 5,6,7,8-THF)
R = 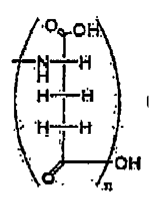 (polyglutamate de l'acide tétrahydroptézoïque)
**caractérisé en ce que**
- on ajoute à un mélange d'un composé de la formule I, un agent antioxydant et un agent donnant un radical formyle, dans un récipient de réaction, une solution tampon aqueuse ayant un pH allant de 6,5 à 7,5,
- on fait passer un gaz inerte dans la suspension obtenue,
- puis on ajoute goutte à goutte ou par fractions, sous agitation stationnaire, une solution aqueuse d'une base, jusqu'à ce qu'il se forme une solution limpide, qui présente un pH allant de 6,5 à 8,3,
- on ajoute alors une glutamate-formyle transférase monofonctionnelle recombinée, abrégée rGFT, qui ne présente que la fonction de fromimino-(formyle)-transférase, à savoir l'enzyme EC 2.1.2.5, dans une solution tampon aqueuse définie ci-dessus,
- on augmente la température dans un intervalle allant de 30°C à 45°C et on fait réagir le mélange réactionnel jusqu'à ce qu'au moins 95% du transfert de formyle ait été réalisé depuis l'agent donnant un radical formyle sur le composé I,
- on refroidit alors le mélange réactionnel à une température allant de 0°C à 5°C,
- on ajoute goutte à goutte, une solution aqueuse d'un acide minéral, jusqu'à ce que l'on ait atteint un pH allant de 2,7 à 3,1, où le composé brut de formule II : 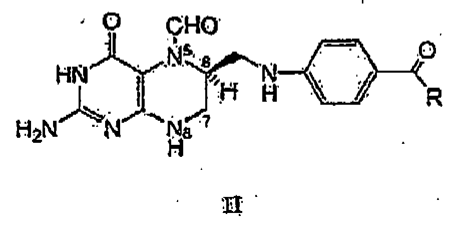
où R est le même radical que dans la formule I, précipite,
- on poursuit l'agitation pendant au moins 1 heure pour compléter la précipitation ci-dessus, et
- le composé brut de formule II est obtenu.

2. Procédé selon la revendication 1, **caractérisé en ce que** le composé de formule (I) est l'acide (6S)-5,6,7,8-tétrahydrofolique, abrégé (6S)-THF.

3. Procédé selon l'une des revendications 1 à 2, **caractérisé en ce que** l'on dispose d'abord le composé de formule I sous forme solide dans le récipient de réaction mentionné,
puis on ajoute l'agent antioxydant, choisi parmi le groupe consistant en l'acide ascorbique, le mercaptoéthanol et le dithiotréithol, et
on ajoute l'agent donnant un radical formyle, de préférence choisi parmi le groupe consistant en l'acide N-formyl-(L)-glutamique, la N-formyl-(L)-glutamine, la N-formyl-(L)-isoglutamine et l'acide N-formyl-(L)-aspartique, ou leurs sels solubles dans l'eau, de préférence les sels de sodium ou les sels de potassium, et
on prépare le mélange à température ambiante et sous un gaz inerte.

4. Procédé selon l'une des revendications 1 à 3, **caractérisé en ce que** la solution tampon aqueuse est un tampon phosphate ayant un pH allant de 6,5 à 7,5, de préférence un tampon phosphate de sodium ou de potassium 10 mM à 1 M, de préférence 100 mM.

5. Procédé selon l'une des revendications 1 à 4, **caractérisé en ce que** la solution aqueuse d'une base est une solution à 20 % de NaOH ou une solution à 20 % de KOH.

6. Procédé selon l'une des revendications 1 à 5, **caractérisé en ce que** la solution limpide, qui est obtenue après addition de la base, présente un pH allant de 6,5 à 6,8.

7. Procédé selon l'une des revendications 1 à 6, **caractérisé en ce qu'**après l'addition de l'enzyme, la température est augmentée dans un intervalle allant de 37°C à 42°C.

8. Procédé selon l'une des revendications 1 à 7, **caractérisé en ce qu'**après addition de l'enzyme, on laisse réagir les composants pendant au moins 8 heures, de préférence pendant 1 à 5 jours.

9. Procédé selon l'une des revendications 1 à 8, **caractérisé en ce que** l'acide minéral est HCl, de préférence HCl à 18 %, ou H₂SO₄.

10. Procédé selon l'une des revendications 1 à 9, **caractérisé en ce que** l'agitation pour compléter la précipitation du composé brut de formule II est réalisée pendant au moins 2 heures.

11. Procédé selon l'une des revendications 1 à 10, **caractérisé en ce que** le composé brut de formule II est obtenu par filtration ou par centrifugation.

12. Procédé selon l'une des revendications 1 à 11, **caractérisé en ce que** le rapport molaire entre l'agent donnant un radical formyle et le composé de la formule I pendant la réaction de formylation est maintenu dans l'intervalle allant de 2:1 à 10: 1, en particulier de 5:1 à 3:1.

13. Procédé selon l'une des revendications 1 à 12, **caractérisé en ce que** le rapport molaire entre l'agent antioxydant et le composé de la formule I se situe dans l'intervalle allant de 1:1 à 5:1, en particulier de 2:1 à 3:1.

14. Procédé selon l'une des revendications 1 à 13, **caractérisé en ce que** l'on formyle 1 mM à 200 mM de (6S)-THF avec une quantité de 0,01 à 0,1 U/ml, en particulier de 0,03 à 0,04 U/ml d'enzyme, en une période allant de 24 heures à 48 heures, à une température allant de 37°C à 42°C et à un pH allant de 6,5 à 6,8.

15. Procédé selon l'une des revendications 1 à 14, **caractérisé en ce que** la purification du composé brut de formule II comprend la remise en suspension du mélange brut dans l'eau froide et à refiltration.

16. Procédé selon l'une des revendications 1 à 15, **caractérisé en ce que** l'ADN génomique, qui code l'enzyme monofonctionnelle recombinée citée, est isolé d'un microorganisme, en particulier de la famille des *Streptocaccaceae*, de préférence de *S. pyogenes,* par exemple de la souche ATCC 19615, ou d'une souche mutante avec la même fonction, ou est préparé chimiquement.

17. Procédé selon l'une des revendications 1 à 16, **caractérisé en ce que** l'enzyme monofonctionnelle recombinée citée est présente sous une forme immobilisée sur un support, par exemple sur une résine activée époxy.

18. Utilisation d'une glutamate-formyle transférase monofonctionnelle recombinée, qui ne présente que la fonction de fromimino-(formyle)-transférase, à savoir l'enzyme EC 2.1.2.5, pour la N(5)-formylation régiosélective de l'acide (6S)-5,6,7,8-tétrahydroptéroique et de ses dérivés, qui sont définis par la formule (I) : 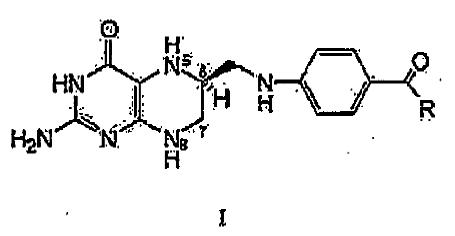 R = OH
R = (L)-glutamyle (= 5,6,7,8-THF) R = 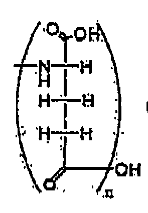 (polyglutamate de l'acide tétrahydroptéroique).
